# FASCICULE DE BREVET EUROPEEN

(11) **EP 2 213 786 B1**
(45) Date de publication et mention de la délivrance du brevet: **09.05.2012**
(21) Numéro de dépôt: 10305095.1
(22) Date de dépôt: 28.01.2010
(51) Int. Cl.: D06M 16/00, D06M 15/19, D06M 10/10, A61L 15/22, A61L 15/44, A61L 15/46, A61M 35/00

(54) **Matériau filamenteux ou fibreux imprégné de substances actives**
Faser- oder Filamentmaterial impregniert mit Wirkstoffen
Filamentary or fibrous material impregnated with active substances

(30) Priorité: 28.01.2009 FR 0950512
(43) Date de publication de la demande: 04.08.2010
(73) Titulaire: Avelana Société Nouvelle, 09300 Villeneuve d'Olmes (FR)
(72) Inventeur: Foropon, Valérie, 09300 SAINT JEAN D'AIGUES VIVES (FR)
(74) Mandataire: Colas, Jean-Pierre

(56) Documents cités:
- EP-A- 0 225 280
- EP-A- 1 359 247
- WO-A-00/12170
- FR-A- 1 015 592
- US-A- 4 514 461
- US-B1- 6 326 015

## Description

La présente invention concerne un matériau filamenteux ou fibreux formé d'un fil, filament ou fibre ou d'assemblages ou de constructions de fils ou de fibres, notamment tissu, tricot, non tissé, nappe textile, contenant une substance active, destinée à être libérée progressivement par contact sur une structure vivante et éventuellement pénétrer la peau. L'invention concerne également le procédé d'obtention d'un tel matériau.

Différentes techniques ont été utilisées pour la conception de fils ou textiles imprégnés de substances actives.

On peut par exemple citer des méthodes d'imprégnation simple, par trempage ou pulvérisation du produit sur le substrat, pour lesquelles le principe actif est directement appliqué en surface du textile. Cette méthode présente l'inconvénient d'une perte importante de principe actif lors des lavages.

D'autres méthodes mettant en oeuvre la micro-encapsulation ont été également largement décrites. Les microcapsules permettent une protection du principe actif et sa libération par rupture de la paroi de la microcapsule. Par conséquent, le principe actif est protégé lors des lavages du substrat à condition que la membrane de la microcapsule résiste aux contraintes mécaniques et chimiques des lavages. Les microcapsules peuvent être directement imprégnées ou greffées sur le substrat. Cette méthodologie ne permet cependant pas de « charger » suffisamment le substrat en principe actif en raison de la faible taille des microcapsules et donc de leur faible capacité à contenir le principe actif.

Wo et al (US 4,514,461) ont développé une méthode d'imprégnation d'un substrat avec des microcapsules de polymère, insolubles dans l'eau, contenant une huile essentielle. Les capsules sont mises en solution avec un liant de type latex acrylique liquide. La solution ainsi obtenue peut être pulvérisée sur le substrat. Un deuxième procédé vise à tremper le substrat dans la solution de microparticules et liant, puis à passer le substrat entre deux cylindres chauffés, ce qui permet d'assurer une pénétration profonde et uniforme.

Copete Vidal et al (US2005/0150056) ont également développé une méthode de fixation de ces microcapsules sur le substrat, en utilisant un liant. Les liants choisis parmi les composés polymériques silicones, les poly(méth)acrylates, etc., permettent une adhésion plus ferme des microcapsules au substrat et donc une résistance accrue au lavage. L'application peut être réalisée par imprégnation du substrat par une solution aqueuse contenant les principes actifs encapsulés et le(s) liant(s) ; la solution peut également être appliquée par pression sur le substrat.

D'autres méthodes ont également été décrites. Notamment, la demande de brevet EP1002810 décrit une émulsion de polymère contenant des particules de polymères composées de chitosan et d'un polymère d'acide organique permettant d'obtenir un revêtement continu (film) ou discontinu (particules) à la surface des fibres. L'utilisation d'un liant est également décrite comme préférable pour la résistance au lavage.

Ces solutions ont pour principal inconvénient de nécessiter la préparation de microcapsules ou de microparticules spécifiques, qui plus est de dimensions suffisamment réduites pour ne pas modifier la main du tissu (touché et tombé du tissu) lorsqu'une proportion d'entre elles reste à la surface du tissu. Ces solutions sont en outre coûteuses et peu efficaces en raison de la faible contenance des microcapsules.

Un autre procédé décrit dans WO 00/12170 concerne l'application d'une couche gélatineuse sur un substrat. Ladite couche gélatineuse comprend un principe actif, une huile plastifiante et un bloc copolymère de haute viscosité (supérieure ou égale à 1800 cps à 25°C). Cette méthode présente l'inconvénient de ne permettre qu'un dépôt en surface d'une couche gélatineuse relativement épaisse et ne peut donc pas être appliquée à la production d'articles d'habillement ou autres articles pour lesquels il n'est pas souhaitable de modifier de manière substantielle la main du tissu.

La présente invention a pour objectif de remédier aux inconvénients de l'art antérieur (notamment quantités importantes, durabilité) en proposant un matériau filamenteux ou fibreux formé d'un fil, filament ou fibre ou d'un assemblage de fils, de filaments ou de fibres ou d'une structure à base de fils, filaments ou fibres en une matière organique naturelle, artificielle, synthétique ou minérale incorporant un principe actif cosmétique, pharmaceutique ou phytosanitaire de manière durable mais permettant sa libération sur une structure vivante par contact.

Un autre objectif encore de l'invention est de permettre l'incorporation de principe actif dans un tissu, un non-tissé, une nappe textile, etc., sans en modifier les propriétés textiles : touché, élasticité, extensibilité, drapé, etc.

Un autre objectif encore de l'invention est d'incorporer des quantités importantes de principe actif, et cela de manière durable.

La présente invention concerne un matériau filamenteux ou fibreux formé d'un fil, d'un filament, d'une fibre, ou d'un assemblage de fils, de filaments et/ou de fibres, en une matière organique naturelle, artificielle, synthétique ou minérale, et imprégné à coeur d'une matrice polymérisée et/ou réticulée dans laquelle une huile, liquide à la température corporelle, est présente à l'état dispersé, cette huile étant apte à être libérée lorsque le matériau est en contact d'une structure vivante.

Par « à l'état dispersé », on entend au sens de l'invention que l'huile est directement dispersée dans la matrice polymérisée. Par exemple, elle n'est pas incluse dans une structure telle qu'une microcapsule, microsphère ou microparticule comportant une membrane ou paroi encapsulant l'huile. L'huile est simplement dispersée sous forme de gouttelettes dans la masse de la matrice. L'huile est directement au contact de la matrice.

Par « structure vivante », on entend homme, animal et végétaux. On entend plus particulièrement une surface corporelle de l'homme ou animal ou une surface extérieure d'un végétal. On peut ainsi citer pour l'homme la peau ou les cheveux et pour les animaux, la peau, le plumage ou le pelage. Pour un végétal, on peut citer la tige, le tronc, les feuilles, les racines.

Cette huile apporte des propriétés intéressantes pour la structure vivante soit par elle-même, soit par l'apport progressif d'un ou plusieurs principes actifs, ou par une combinaison des deux. L'huile et/ou le principe actif sont libérés progressivement par contact et éventuellement par lipophilie. Selon la nature de l'huile et/ou du principe actif, une pénétration de la peau chez l'homme et l'animal ou une pénétration dans le végétal peut être obtenue.

Par définition, on entend par « imprégnation à coeur » par la matrice une imprégnation profonde, entre les fils, fibres ou filaments constitutifs du matériau, et/ou à l'intérieur des fils, fibres ou filaments lorsque la matière constitutive des fils, fibres ou filaments est capable d'absorber une certaine quantité d'huile. Par définition, la matrice est polymérisée et/ou réticulée après imprégnation. L'imprégnation se fait à l'aide de la matrice polymérisable et/ou réticulable dans laquelle l'huile a été dispersée.

Par « température corporelle », on entend la température présente à la surface de l'organisme vivant. Pour l'homme et les animaux, cette température peut être typiquement comprise entre 20 et 40 °C, notamment entre 28 et 37 °C. Pour les végétaux, cette température dépend notamment de la température de l'environnement dans laquelle se trouve ce végétal. Le lien entre température corporelle et état liquide de l'huile est clair et accessible pour l'homme du métier.

La matrice est obtenue à partir de monomères et/ou de polymères.

Une matrice polymérisée est une matrice formée par polymérisation d'un monomère, d'un mélange de monomères, d'un polymère ou d'un mélange de polymères. Une matrice réticulée est une matrice formée d'un matériau polymère dont les macromolécules le constituant sont liées entre elles par des liaisons obtenues par réticulation. La matrice peut aussi être polymérisée et réticulée.

Selon une caractéristique de l'invention le matériau peut être constitué de plusieurs filaments ou de plusieurs fibres.

On entend par filament un élément unitaire de très grande longueur, mince, dit continu, constitutif d'un fil.

On entend par fibre un élément unitaire de courte longueur, mince, dit discontinu, utilisable pour la fabrication de fils ou de non-tissés.

Les fibres ou filaments selon l'invention sont d'origine naturelle, synthétique, artificielle ou minérale. Ils sont avantageusement lipophiles.

On entend par fibres ou filaments naturels, des filaments ou fibres d'origine animale ou végétale. Les fibres ou filaments naturels peuvent notamment être choisis parmi coton, lin, laine, chanvre, ramie, soie, jute ou tout autre type de fibre ou filament naturel.

On entend par fibres ou filaments artificiels, des fibres ou filaments réalisés par l'Homme à partir d'éléments naturels. Les fibres et filaments artificiels sont notamment choisis parmi viscose, cupro, fibre de maïs, chitine ou tout autre type de fibre ou filament artificiel.

On entend par fibres ou filaments synthétiques, des fibres ou filaments réalisés en tout point par l'Homme grâce à la chimie de synthèse. Les fibres et filaments synthétiques sont notamment choisis parmi polyamide, polyester, chlorofibres, acrylique, polypropylène ou tout autre type de fibre ou filament synthétique.

Les fibres ou filaments minéraux lipophiles sont notamment choisis parmi tourbe ou toute autre fibre ou filament d'origine minérale.

Selon une caractéristique de l'invention, le matériau peut être choisi parmi les tissus, les tricots, les non-tissés, les nappes textiles, les fils, à savoir les fils continus (fils formés de plusieurs filaments ou fils formés d'un seul filament), les filés (fils formés de fibres), etc. Les tissus, les tricots, non tissés, nappes textiles sont formés de l'assemblage de fils continus pluri- ou monofilaments, de filés et/ou de fibres.

Selon une caractéristique de l'invention, avant imprégnation, le matériau a une prise d'huile (absorption d'huile) supérieure ou égale à 0,2 ml d'huile pour 100 g de matériau. La prise d'huile est de préférence comprise entre 0,2 et 100 ml d'huile pour 100 g de matériau. Cette prise d'huile est mesurée comme décrit plus loin.

Selon une première variante de l'invention, l'huile est elle-même en tout ou partie un principe actif cosmétique, pharmaceutique ou phytosanitaire, c'est-à-dire qu'elle possède ou qu'elle comprend au moins une huile ayant des propriétés cosmétiques, pharmaceutiques ou phytosanitaire.

L'huile peut être choisie parmi tout type d'huile, végétale, animale, minérale ou synthétique. Elle est préférentiellement choisie parmi les huiles végétales et les huiles animales.

L'huile végétale utilisée pourra être de l'huile d'arachide, d'argan, de bourrache, de colza, de caméline, de germe de blé, de jojoba, de noisette, de noix, de lin, de maïs, d'olive, de palme, de pépins de raisin, de ricin, de soja, de tournesol, ou toute autre huile d'origine végétale ou une huile essentielle parmi l'huile essentielle de bergamote, de camomille, de carotte, de cèdre, de ciste, de citron, de citronnelle, d'eucalyptus, de cyprès, de géranium, de lavande, de marjolaine, de menthe, de myrte, de romarin ou toute autre huile essentielle d'origine végétale. Il peut s'agir de mélanges de deux ou plus de ces huiles.

L'huile animale utilisée pourra être une huile de poisson, de baleine, de cachalot, de foie de morue ou toute autre huile d'origine animale. Il peut s'agir de mélanges de deux ou plus de ces huiles.

Différents types d'huiles peuvent aussi être mélangés.

Selon une autre variante de l'invention, l'huile comporte un ou plusieurs principes actifs cosmétiques, pharmaceutiques (e.g. médicaments, antimicrobiens) ou phytosanitaires.

Ces principes actifs vont notamment permettre, chez l'Homme et l'animal, par contact avec les parties superficielles du corps, de nettoyer, parfumer, modifier l'aspect, protéger, maintenir en bon état, corriger les odeurs corporelles, soigner, soulager, nourrir, chauffer, rafraîchir, etc.

Ces principes actifs peuvent, chez les végétaux, par contact avec les parties superficielles, traiter, protéger, nourrir, modifier l'aspect ou la couleur, nettoyer, parfumer, maintenir en bon état, etc.

Dans un mode de réalisation de l'invention, l'huile comporte un principe actif solide dispersé dans l'huile (forme solide dans huile) ou dans une phase aqueuse (eau par exemple) ou dans une phase alcoolique, cette phase aqueuse ou alcoolique étant elle-même dispersée dans l'huile (forme solide dans eau dans huile, ou encore solide dans alcool dans huile).

Dans un autre mode de réalisation de l'invention, l'huile comporte un principe actif dissout dans l'huile.

Dans un autre mode de réalisation de l'invention, l'huile comporte un principe actif en solution aqueuse ou alcoolique, dispersé ou en émulsion dans l'huile. On peut donc avoir l'une des formes suivantes : eau dans l'huile, alcool dans huile.

La matrice polymérisable et/ou réticulable imprègne le matériau et, après polymérisation et/ou réticulation, forme un réseau continu ou discontinu au sein de celui-ci. La matrice comprend ou est formée d'une matière polymérisée et/ou réticulée choisie parmi les amines, les amides, les imines, les acrylates, les polyuréthanes, les composés vinyliques, les hydroxyles, les carboxyles, les carbonyles et les métacrylates, seuls ou en mélange. Des détails concernant cette matrice sont donnés plus loin à propos du procédé de préparation.

On peut citer à titre d'exemple, sans être limitatif, une matrice obtenue à partir d'une résine formée d'amine tertiaire réactive, telle que par exemple l'Ebecryl^{®}P115 commercialisée par CYTEC.

La présente invention concerne également des articles tissés, tricotés ou non tissés comportant le matériau tel que défini précédemment. Ces articles peuvent avoir une application chez l'Homme, les animaux et les végétaux.

L'invention concerne notamment des articles tissés ou tricotés à l'aide exclusive ou non de fils continus ou de filés selon l'invention. Elle concerne aussi des articles tissés ou tricotés incorporant un matériau selon l'invention, notamment un tissu, un non-tissé ou une nappe textile selon l'invention. Elle concerne aussi des articles tricotés ou tissés incorporant un ou plusieurs fils continus ou filés selon l'invention.

L'invention concerne aussi des articles non-tissés réalisés à l'aide exclusive ou non de fibres selon l'invention.

Ledit article est notamment un article d'habillement, de décoration, d'ameublement, de soin (e.g. article d'orthopédie, patch, pansement), de bien-être (e.g. lingette) ou tout article pouvant être appliqué de manière superficielle sur l'homme, l'animal ou le végétal. Par exemple, chez l'homme, il peut s'agir de tout article pouvant être appliqué au contact de la peau (l'article peut être apposé, collé, enfilé, etc. et l'homme peut être installé sur ledit article).

On entend par « article de décoration ou d'ameublement » des meubles ou articles de décoration, notamment de type chaise, lit, canapé, fauteuil de soin, table de massage, panier pour animaux... sur lequel l'homme ou l'animal peuvent s'installer, notamment s'asseoir, s'allonger.... L'article étant recouvert de la matrice selon l'invention, le(s) principe(s) actif(s) sont libéré(s) au contact des parties superficielles de l'homme ou de l'animal.

A titre d'exemple on peut citer un fauteuil de soin recouvert de la matrice selon l'invention et qui libère au contact de la peau de l'homme, installé sur ledit fauteuil, le(s) principe(s) actif(s).

L'invention concerne également le procédé d'obtention d'un matériau filamenteux ou fibreux formé d'un fil, filament ou fibre ou d'un assemblage de fils, de filaments et/ou de fibres, en une matière organique naturelle, artificielle, synthétique ou minérale, caractérisé en ce que :
a) on mélange une huile liquide à la température corporelle et une matrice polymérisable et/ou réticulable destinée à former une matrice polymérisée et/ou réticulée,
b) on imprègne le matériau du mélange de l'étape a),
c) on élimine si nécessaire le surplus de mélange,
d) on polymérise et/ou réticule pour former la matrice polymérisée et/ou réticulée,
ce grâce à quoi on obtient un matériau filamenteux ou fibreux conforme à l'invention, imprégné à coeur d'une matrice polymérisée et/ou réticulée dans laquelle une huile est présente à l'état dispersé.

La matrice polymérisable et/ou réticulable est de préférence une préparation liquide de monomères et/ou de polymères. Il peut s'agir d'une dispersion de monomères et/ou de polymères, d'une solution de monomères et/ou de polymères ou encore de manière préférée de monomères ou polymères sous forme liquide à l'état pur ou substantiellement pur. S'agissant d'une dispersion, les monomères et/ou polymères sont à l'état divisé dans un liquide, notamment un liquide organique ou aqueux, y compris l'eau, en présence éventuelle d'additifs. S'agissant d'une solution, les monomères et/ou polymères sont dissouts dans un liquide, notamment un liquide organique ou aqueux, y compris l'eau et l'huile, en présence éventuelle d'additifs, dont certains seront décrits plus loin.

Selon une caractéristique de l'invention, le mélange huile + matrice se présente sous forme de dispersion ou d'émulsion dans un liquide, notamment un liquide organique ou aqueux, y compris de l'eau.

Par exemple, l'huile forme avec la matrice une dispersion ou une émulsion répondant à la formule suivante :
- entre 0,5 et 50% de principe(s) d'actif(s) + huile ou huile seule lorsqu'elle constitue le principe actif ; le principe actif peut être par exemple sous forme huileuse, sous forme eau dans l'huile, sous forme alcool dans huile, sous forme solide dans huile, sous forme solide dans eau dans huile, ou encore solide dans alcool dans huile,
- entre 5 et 99,5% d'une matrice polymérisable et/ou réticulable,
- entre 0 et 90% d'eau.

Le matériau peut comprendre en outre des additifs tels que des agents dispersants et/ou mouillants et/ou homogénéisants, notamment des détergents, des tensio-actifs ioniques, des tensio-actifs amphotères, des tensio-actifs non ioniques, des préparations alcooliques, des latex acryliques, etc ; des photoinitiateurs, des agents de protection, notamment antioxydants, protecteurs thermiques, etc. ; des produits auxiliaires, notamment agents antistatiques, adoucissants, agents hydrofuges, agents hydrophiles, agents oléophiles, agents oléofuges, pigments, oxydes métalliques, etc.

A titre d'exemple de tensio-actif, on peut citer, sans être limitatif, les polyarylphénol éthoxyle, tel que celui ayant le n° CAS : 99734-09-5 ; un tel tensio-actif est commercialisé sous la dénomination Soprophor BSU par RHODIA CHIMIE.

Selon une variante de l'invention, les étapes b) et c) comprennent une étape de foulardage ou méthode analogue, qui permet de réaliser l'imprégnation et l'élimination du surplus de mélange matrice + huile, si nécessaire. Comme méthode analogue, on peut citer l'application par racle, notamment racle en l'air.

Selon une autre variante de l'invention, l'étape b) comprend une étape de pulvérisation ou une étape de dépose à l'aide de rouleaux type impression ou mille points.

Selon une caractéristique de l'invention, l'étape d) de polymérisation et/ou réticulation est réalisée par traitement aux rayons ionisants, notamment de types rayonnements ultraviolets ou rayonnements gamma, soit par traitement thermique, soit par traitement thermique et rayons ionisants.

Le temps de traitement aux rayons ionisants peut être compris entre quelques secondes et 3 minutes. De manière avantageuse, ce traitement se fait en présence d'au moins un photoinitiateur, notamment la benzophénone.

Le traitement en présence d'au moins un photoinitiateur peut être couplé à un autre initiateur thermique s'activant sous l'effet de la température. Un tel initiateur peut être le peroxyde de méthyle éthyle cétone.

Le système catalytique ainsi obtenu peut en outre comprendre d'autres photoinitiateurs notamment choisi parmi les alphahydroxycétones ou tout autre mélange de différents photoinitiateurs.

Dans le cas d'utilisation de rayonnements ionisants de type rayons gamma, l'utilisation d'un photoinitiateur n'est pas nécessaire.

Selon une caractéristique de l'invention, la polymérisation et /ou réticulation par traitement thermique pourra être réalisée dans une enceinte à air chaud notamment de type rame pour textile ou séchoir fil ou à l'aide de lampes infrarouges ou par tout autre moyen permettant d'atteindre des températures adéquates, par exemple supérieures à 120°C.

Selon une caractéristique de l'invention les rayonnements ionisants et le traitement thermique peuvent être utilisés conjointement pour la polymérisation et/ou réticulation. Ces deux traitements peuvent alors être effectués dans un ordre non établi, à savoir rayonnement ionisant, puis traitement thermique, traitement thermique, puis rayonnement ionisant ou rayonnement ionisant et traitement thermique au même instant.

Les traitements ainsi définis permettent de créer un véritable réseau au sein du matériau, qui piège le ou les principes actifs, qui évite une libération trop rapide d'actif(s), notamment lors des lavages, tout en permettant une biocompatibilité et une biodistribution du ou des actifs devant être libérés progressivement.

La présente invention concerne également un matériau filamenteux ou fibreux formé d'un fil, d'un filament, d'une fibre, ou d'un assemblage de fils, de filaments et/ou de fibres, en une matière organique naturelle, artificielle, synthétique ou minérale, et imprégné à coeur d'une matrice polymérisée et/ou réticulée dans laquelle une huile, liquide à la température corporelle, est présente à l'état dispersé, cette huile étant apte à être libérée lorsque le matériau est en contact d'une structure vivante, ce matériau étant susceptible d'être obtenu par le procédé de l'invention.

L'invention va maintenant être décrite à l'aide d'exemples non limitatifs.

### EXEMPLES :

### 1. Mesure de la prise d'huile :

La prise d'huile est une technique permettant de vérifier le taux d'imprégnation d'huile dans le matériau. La prise d'huile est déterminée par adaptation de la norme internationale ISO 787/5 (« *Méthodes générales d'essai des pigments et matières de charge* », *partie 5 : « détermination de la prise d'huile* ») en utilisant une huile de lin selon la norme ISO 150 concernant les huiles de lin brutes raffinées ou cuites, pour peintures et vernis. On définit la prise d'huile comme étant la quantité d'huile de lin raffinée qui est absorbée dans des conditions définies par un échantillon de matériau. Le réactif utilisé en référence est une huile de lin raffinée, conforme aux spécifications de la norme ISO 150, ayant un indice acide de 5 à 7 mg de KOH par gramme.

La prise d'huile, nécessite deux déterminations, et est effectuée comme suit:
1. on prélève un échantillon représentatif du produit,
2. on pèse la quantité appropriée d'échantillon (ce qui donne l'éprouvette), correspondant à sa prise d'huile présumée comme suit :

| Prise d'huile présumée (ml/100g) | Masse de l'éprouvette (g) |
|---|---|
| Moins de 10 | 20 |
| 10 à 30 | 10 |
| 30 à 50 | 5 |
| 50 à 80 | 2 |
| Plus de 80 | 1 |

3. on effectue la détermination. Pour cela on place l'éprouvette sur une plaque de verre dépoli ou de marbre d'au moins 300 mm x 400 mm. On ajoute lentement l'huile de lin, à raison d'une goutte à la fois, au moyen d'une burette de 10 ml de capacité conforme aux spécifications de l'ISO/R 385. Après chaque addition on incorpore l'huile dans le produit avec un couteau à palette, à lame biseauté en acier, dont les dimensions sont de l'ordre de 140 à 150 mm de longueur, de 20 à 25 mm de largeur à sa partie la plus large et au moins 12,5 mm de largeur à sa partie la plus étroite. On continue d'ajouter l'huile à cette cadence. Lorsque l'huile pénètre plus difficilement dans le produit on fait suivre chaque addition d'huile d'une trituration énergétique avec le couteau. Lorsqu'une goutte d'huile reste à la surface du produit ou est détectable sur la plaque, l'addition d'huile est stoppée. On note, d'après la burette, la quantité d'huile utilisée. L'ensemble de l'opération dure de 20 à 25 minutes. Durant ce temps, la masse totale du produit doit être travaillée par l'opérateur avec un effort maximal.

Les résultats de la prise d'huile sont exprimés en millilitres d'huile par 100 mg de produit ou en gramme d'huile par 100 g de produit.

### 2. Exemple 1

On dispose d'un bain de foulardage de type solution aqueuse dont la composition est la suivante : 40g/l d'Ebecryl^{®}P115 (amine tertiaire réactive), 10g/l d'huile de caméline (actif), 0,5g/l de Soprophor BSU (polyarylphénol éthoxyle, n° CAS : 99734-09-5) (émulsifiant) et 4g/l de benzophénone (photoiniateur) ; et d'un tissu tissé.

Le tissu a une prise d'huile supérieure à 0,2 ml d'huile pour 100 g de matériau constitutifs du tissu.

Le tissu est foulardé, c'est-à-dire qu'on imprègne le tissu de la solution aqueuse par trempage de celui-ci dans le bain de foulardage. Le tissu est ensuite passé entre deux rouleaux exprimeurs ayant pour rôle de contrôler le pourcentage de dépôt de la solution aqueuse sur le tissu. Ces opérations sont réalisées 4 fois.
Les paramètres du foulard (vitesse de défilement du tissu, pression des rouleaux exprimeurs) sont déterminés pour avoir, pour la première imprégnation, un taux d'emport de la solution aqueuse sur le tissu égal à 80% du poids du tissu. Ces paramètres sont ensuite conservés pour les trois autres passages au foulard.
Le tissu est soumis après chaque imprégnation à des rayonnements UV, puis après le quatrième passage sous les rayonnements, le tissu est séché en rame (séchage par air chaud) à 160°C.

## Revendications

1. Matériau filamenteux ou fibreux, formé d'un fil, filament ou fibre ou d'un assemblage de fils, de filaments et/ou de fibres, en une matière organique naturelle, artificielle, synthétique ou minérale, **caractérisé en ce qu'**il est imprégné à coeur d'une matrice polymérisée et/ou réticulée après imprégnation dans laquelle une huile, liquide à la température corporelle, est présente directement à l'état dispersé, cette huile étant apte à être libérée progressivement, éventuellement par lipophilie, lorsque le matériau est au contact d'une structure vivante.

2. Matériau selon la revendication 1, **caractérisé en ce qu'**il est choisi parmi : tissu, tricot, non-tissé, nappe textile, fil continu, filé.

3. Matériau selon la revendication 1 ou 2, **caractérisé en ce que**, avant imprégnation, le matériau a une prise d'huile supérieure ou égale à 0,2 ml d'huile pour 100 g de matériau, la prise d'huile est déterminée par adaptation de la norme internationale ISO 787/5 (« *Méthodes générales d'essai des pigments et matiéres de charge* », *partie 5 : « détermination de la prise d'huile*») en utilisant une huile de lin selon la norme ISO 150 concernant les huiles de lin brutes raffinées ou cuites, pour peintures et vernis.

4. Matériau se on l'une quelconque des revendications précédentes, **caractérisé en ce que** l'huile est un actif cosmétique ou pharmaceutique ou phytosanitaire.

5. Matériau se:on l'une quelconque des revendications précédentes, **caractérisé en ce que** l'huile comporte un principe actif cosmétique ou pharmaceutique ou phytosanitaire.

6. Matériau selon la revendication 5, **caractérisé en ce que** l'huile comporte un principe actif solide dispersé ou dissout dans l'huile.

7. Matériau selon la revendication 5, **caractérisé en ce que** l'huile comporte un principe actif en phase aqueuse dispersé ou en émulsion dans l'huile.

8. Matériau selon l'une quelconque des revendications précédentes, **caractérisé en ce que** l'huile est choisie parmi les huiles végétales, animales, minérales ou synthétiques.

9. Matériau selon l'une quelconque des revendications précédentes, **caractérisé en ce que** la matrice comprend ou est formée d'une matière réticulée et/ou polymérisée choisie parmi les amines, les amides, les imines, les acrylates, les polyuréthanes, les composés vinyliques, les hydroxyles, les carboxyles, les carbonyles et les métacrylates seuls ou en mélange.

10. Article tissé tricoté ou non tissé comportant un matériau selon l'une quelconque des revendications précédentes.

11. Article selon la revendication 10, **caractérisé en ce qu'**il s'agit d'un article d'habillement, de décoration, d'ameublement, de soin, de bien-être ou tout article pouvant être appliqué de manière superficielle sur l'homme, l'animal ou le végétal.

12. Procédé de fabrication d'un matériau filamenteux ou fibreux formé d'un fil, filament ou libre ou d'un assemblage de fils, de filaments et/ou de fibres, en une matière organique naturelle, artificielle, synthétique ou minérale, **caractérisé en ce que** :
a. on mélange une huile liquide à la température corporelle et une matrice polymérisable et/ou réticulable destinée à former une matrice par polymérisation et/ou réticulation,
b. on imprègne le matériau avec le mélange de l'étape a),
c. on élimines nécessaire le surplus de mélange,
d. on polymérise et/ou réticule pour former la matrice polymérisée et/ou réticulée, ce grâce à quoi on obtient un matériau filamenteux ou fibreux imprégné à coeur d'une matrice polymérisée et/ou réticulée dans laquelle une huile est présente à l'état dispersé.

13. Procédé selon la revendication 12, **caractérisé en ce que** les étapes b) et c) sont réalisées par foulardage.

14. Procédé selon la revendication 12, **caractérisé en ce que** l'étape b) comprend une pulvérisation de la solution.

15. Procédé selon l'une des revendications 12 à 14, **caractérisé en ce que** l'étape d) est réalisée par rayonnement ionisant et/ou par traitement thermique.

## Claims

1. A filamentous or fibrous material formed by a thread, filament or fibre, or an assembly of threads, filaments and/or fibres made of a natural, artificial, synthetic or mineral organic material, **characterised in that** it is impregnated to the core with a polymerised and/or crosslinked matrix after impregnation in which an oil, liquid at body temperature, is present directly in the dispersed state, this oil being able to be released progressively, possibly by lipophily, when the material is in contact with a living structure.

2. The material according to Claim 1, **characterised in that** it is chosen from: woven, knitted or non-woven fabric, textile cloth, continuous thread, spun yarn.

3. The material according to Claim 1 or 2, **characterised in that**, before impregnation, the material has an oil absorption greater than or equal to 0.2 ml of oil per 100 g of material, the oil absorption is determined by adaptation of international standard ISO 787/5 ("*General methods for testing pigments and fillers*", *part* 5: "*determination of oil absorption*") using a linseed oil according to standard ISO 150 with regard to crude, refined or boiled linseed oils for paints and varnishes.

4. The material according to any of the preceding claims, **characterised in that** the oil is a cosmetic or pharmaceutical or phytosanitary active principle.

5. The material according to any of the preceding claims, **characterised in that** the oil comprises a cosmetic or pharmaceutical or phytosanitary active principle.

6. The material according to Claim 5, **characterised in that** the oil comprises a solid active principle dispersed or dissolved in oil.

7. The material according to Claim 5, **characterised in that** the oil comprises an active principle in aqueous phase dispersed or in emulsion in the oil.

8. The material according to any of the preceding claims, **characterised in that** the oil is chosen from vegetable, animal, mineral or synthetic oils.

9. The material according to any of the preceding claims, **characterised in that** the matrix comprises or is formed by a crosslinked and/or polymerised material chosen from amines, amides, imines, acrylates, polyurethanes, vinyl compounds, hydroxyls, carboxyls, carbonyls and methacrylates on their own or in a mixture.

10. A woven, knitted or nonwoven item comprising a material according to any of the preceding claims.

11. The item according to Claim 10, **characterised in that** it is a clothing, decoration, furnishing, care or comfort item, or any item that can be applied superficially to a person, animal or plant.

12. A method of producing a filamentous or fibrous material formed by a thread, filament or fibre, or an assembly of threads, filaments and/or fibres made of a natural, artificial, synthetic or mineral organic material, **characterised in that**:
a. an oil liquid at body temperature is mixed with a matrix that can be polymerised and/or crosslinked intended to form a matrix by polymerisation and/or crosslinking,
b. the material is impregnated with the mixture of step a),
c. if necessary the surplus mixture is eliminated,
d. one polymerises and/or crosslinks in order to form the polymerised and/or crosslinked matrix, by means of which one obtains a filamentous or fibrous material impregnated to the core with a polymerised and/or crosslinked matrix in which an oil is present in the dispersed state.

13. The method according to Claim 12, **characterised in that** steps b) and c) are implemented by padding.

14. The method according to Claim 12, **characterised in that** step b) includes spraying of the solution.

15. The method according to any of Claims 12 to 14, **characterised in that** step d) is implemented by ionising radiation and/or by heat treatment.

## Patentansprüche

1. Filament- oder faserartiges Material, das aus einem Faden, einen Filament oder einer Faser oder aus einer Anordnung von Fäden, Filamenten und/oder Fasern aus einem natürlichen organischen, künstlichen, synthetischen oder mineralischen Stoff gebildet ist, **dadurch gekennzeichnet, dass** es mit einem polymerisierten und/oder nach der Imprägnierung vertretzten Grundstoff durchimprägniert ist, in dem ein Öl, das bei Körpertemperatur flüssig ist, direkt im dispergierten Zustand vorhanden ist, wobei dieses Öl allmählich, eventuell durch Lipophilie, freigesetzt werden kann, wenn das Material mit einer lebenden Struktur in Kontakt ist.

2. Material nach Anspruch 1, **dadurch gekennzeichnet, dass** es gewählt ist aus: Gewebe, Trikot, Vlies, Textilcord, ununterbrochener Faden, Gespinst.

3. Material nach Anspruch 1 oder 2, **dadurch gekennzeichnet, dass** vor dem Imprägnieren das Material eine Ölaufnahme größer oder gleich 0,2 ml Öl pro 100 g Material besitzt, wobei die Ölaufnahme durch Übernahme der internationalen Norm ISO 787/5 ("Allgemeine Prüfverfahren für Pigmente und Füllstoffe", Teil 5: "Bestimmung der Ölaufnahme") unter Verwendung eines Leinöls gemäß der Norm ISO 150, die rohe, raffinierte oder gekochte Leinöle für Farben und Lacke betrifft, bestimmt wird.

4. Material nach einem der vorhergehenden Ansprüche, **dadurch gekennzeichnet, dass** das Öl ein kosmetischer oder pharmazeutischer oder phytosanitärer Wirkstoff ist.

5. Material nach einem der vorhergehenden Änsprüche, **dadurch gekennzeichnet, dass** das Öl einen kosmetischen oder pharmazeutischen oder phytosanitären Hauptwirkstoff enthält.

6. Material nach Anspruch 5, **dadurch gekennzeichnet, dass** das Öl einen festen Hauptwirkstoff enthält, der in dem Öl dispergiert oder gelöst ist.

7. Material nach Anspruch 5, **dadurch gekennzeichnet, dass** das Öl einen Hauptwirkstoff in der wässrigen Phase, der in dem Öl dispergiert oder in Emulsion ist, enthält.

8. Material nach einem der vorhergehenden Ansprüche, **dadurch gekennzeichnet, dass** das Öl unter pftanzlichen, tierischen, mineralischen oder synthetischen Ölen gewählt ist.

9. Material nach einem der vorhergehenden Ansprüche, **dadurch gekennzeichnet, dass** der Grundstoff ein vernetztes und/oder polymerisiertes Material enthält oder daraus gebildet ist, das gewählt ist, einzeln oder als Gemisch, aus Aminen, Amiden, Iminen, Acrylaten, Polyurethanen, Vinylverbindungen, Hydroxylen, Carboxylen, Carbonylen und Methacrylaten.

10. Web-, Wirk- oder Vliesartikel, der ein Material nach einem der vorhergehenden Ansprüche enthält.

11. Artikel nach Anspruch 10, **dadurch gekennzeichnet, dass** es sich um einen Bekleidungs-, Dekorations-, Einrichtungs-, Pflege- oder Wellness-Artikel oder um jeden anderen Artikel, der auf die Oberfläche eines Menschen, eines Tiers oder einer Pflanze aufgebracht werden kann, handelt.

12. Verfahren zur Herstellung eines filamentartigen oder faserartigen Materials, das aus einem Faden, einem Filament oder einer Faser oder aus einer Anordnung von Fäden, Filamenten und/oder Fasern aus einem natürlichen organischen, künstlichen, synthetischen oder mineralischen Stoff gebildet ist, **dadurch gekennzeichnet, dass**:
a. ein bei Körpertemperatur flüssiges Öl und ein polymerisierbarer und/oder vernetzbarer Grundstoff, der dazu bestimmt ist, durch Polymerisation und/oder Vernetzung einen Grundstoff zu bilden, gemischt werden,
b. das Material mit dem Gemisch von Schritt a) imprägniert wird,
c. gegebenenfalls das überschüssige Gemisch entfernt wird,
d. eine Polymerisation und/oder eine Vernetzung ausgeführt werden, um den polymerisierten und/oder vernetzten Grundstoff zu bilden, wodurch ein filamentartiges oder faserartiges Material erhalten wird, dessen polymerisierter und/oder vernetzter Grundstoff imprägniert ist und in dem ein Öl im dispergierten Zustand vörhanden ist.

13. Verfahren nach Anspruch 12, **dadurch gekennzeichnet, dass** die Schritte b) und c) durch Walken ausgeführt werden.

14. Verfahren nach Anspruch 12, **dadurch** gekenntzeichnet, dass der Schritt b) eine Pulverisierung der Lösung umfasst.

15. Verfahren nach einem der Ansprüche 12 bis 14, **dadurch gekennzeichnet, dass** der Schritt d) durch ionisierende Strahlung und/oder Wärmebehandlung verwirklicht wird.
